# EUROPEAN PATENT SPECIFICATION

(11) **EP 2 175 855 B1**
(45) Date of publication and mention of the grant of the patent: **10.04.2019**
(21) Application number: 08786003.7
(22) Date of filing: 09.07.2008
(51) Int. Cl.: A61K 31/475, A61K 9/20, A61K 9/48

(54) **STABLE PHARMACEUTICAL COMPOSITION OF A WATER-SOLUBLE VINORELBINE SALT**
STABILE PHARMAZEUTISCHE ZUSAMMENSETZUNG EINES WASSERLÖSLICHEN VINORELBIN-SALZES
COMPOSITION PHARMACEUTIQUE STABLE D'UN SEL DE VINORELBINE SOLUBLE DANS L'EAU

(30) Priority: 11.07.2007 FR 0756425
(43) Date of publication of application: 21.04.2010
(73) Proprietor: Pierre Fabre Médicament, 92100 Boulogne-Billancourt (FR)
(72) Inventor: PAILLARD, Bruno, F-31670 Labege (FR); AVAN, Jean-Louis, F-31290 Villefranche De Lauragais (FR); BOUGARET, Joël, F-31460 Francarville (FR)
(74) Representative: Regimbeau
(86) International application number: PCT/EP2008/058896
(87) International publication number: WO 2009/007389

(56) References cited:
- WO-A-03/101383
- WO-A-2006/069938
- PIERRE FABRE PHARMA: "FACHINFORMATION: NAVELBINE 20 mg/ 30 mg Weichkapseln"[Online] November 2005 (2005-11), pages 1-4, XP002463594 Retrieved from the Internet: URL:http://bronchialkarzinom2007.de/pdf/fi _navelbine_oral.pdf> [retrieved on 2008-01-07]
- ZHOU X J ET AL: "RELATIVE BIOAVAILABILITY OF TWO ORAL FORMULATIONS OF NAVELBINE IN CANCER PATIENTS" BIOPHARMACEUTICS AND DRUG DISPOSITION, WILEY, CHICHESTER, US, vol. 15, no. 7, 1994, pages 577-586, XP009051771 ISSN: 0142-2782
- ROWINSKY E K ET AL: "Pharmacokinetic, bioavailability, and feasibility study of oral vinorelbine in patients with solid tumors" JOURNAL OF CLINICAL ONCOLOGY, GRUNE AND STRATTON, NEW YORK, NY, US, vol. 12, no. 9, September 1994 (1994-09), pages 1754-1763, XP009086445 ISSN: 0732-183X
- RICHARD M LUSH ET AL: "The absolute bioavailability of oral vinorelbine in patients with solid tumors" CANCER CHEMOTHERAPY AND PHARMACOLOGY, SPRINGER-VERLAG, BE, vol. 56, no. 6, 1 December 2005 (2005-12-01), pages 578-584, XP019334242 ISSN: 1432-0843

## Description

The present invention relates to solid and stable pharmaceutical forms of water-soluble derivatives of Vinca alcaloids, namely vinorelbine ditartrate, intended for oral administration.

Anticancer chemotherapy was initially developed by using the intravenous route. Arguments in favor of this administration route are:
- lesser gastro-intestinal toxicity,
- total bioavailability, as well as
- potentially less *inter* and *intra* patient exposure variations than with the oral route.

The intravenous route is however associated with major drawbacks which limit its use: morbidity of venous access, possible complications of the central venous routes (infection, thrombosis), risk of extravasation.

For the last few years, oral forms of anticancer chemotherapy have been increasingly developed because of a real benefit for the patient. Further, pharmaco-economic considerations which become increasingly important in the selection of therapeutic strategies, are also directed toward developments of oral treatments.

Many exploratory studies have been conducted on the possible use of molecules intended for treating cancer and administered orally, whether these are old active ingredients (e.g.: etoposide, cyclophosphamide and idarubicin), novel synthetic derivatives of fluoropyrimidines (e.g.: UFT, capecitabine, S-1), platinum derivatives (e.g. : JM-216) or Vinca alkaloids (e.g.: vinorelbine).

Vinorelbine or 3',4'-didehydro-4'-deoxy-8'-norvincaleukoblastine is a Vinca alkaloid derivative which exerts a cytostatic effect by inhibiting polymerisation of tubulin.

Vinorelbine, and more particularly its salt, vinorelbine ditartrate, is active i.a. in treating non-small-cell lung cancer and breast cancer.

Unfortunately, this salt is particularly unstable in the solid state.

An injectable form was marketed for the first time in 1989 in France. Today it is marketed in the whole world, as a solution to be diluted for perfusion, at a concentration of 10 mg/ml expressed as vinorelbine base and distributed in unit flasks with volumes of 1 and 5 ml.

More recently, an oral formulation of vinorelbine in solution was developed and marketed under the name of Navelbine Oral® (WO 03/101383). It appears as a soft gelatine capsule containing vinorelbine ditartrate in dissolved form and an excipient mixture comprising polyethylene glycol, glycerol, ethanol and water. The average molecular weight of polyethylene glycol is comprised between 200 and 600: these are liquid polyethylene glycols such as Macrogol 400. The unitary dosages expressed as vinorelbine base are comprised between 5 mg and 100 mg and more advantageously are equal to 20 mg, 30 mg, 40 mg and 80 mg.

Oral forms containing vinorelbine ditartrate in a dispersed form in a mixture based on molten polyethylene glycol have also been developed and are the subject of a patent (published under number FR 2 880 274). The unitary dosages expressed as vinorelbine base are comprised between 5 mg and 100 mg, and more advantageously are equal to 20 mg, 30 mg, 40 mg and 80 mg.

Zhou et al. (Biopharmaceutics & Drug Disposition 1994, 15, 577-586) reports that an oral formulation in the form of a capsule containing a solution of Navelbine (vinorelbine) allows a better Navelbine absorption than an oral formulation in the form of a capsule containing Navelbine in powder.

Nevertheless, the development of soft capsules filled with liquid has proved to be difficult. The liquid composition on the one hand should take into account the material of the capsule and avoid degradation of the latter when it has been encapsulated. It should also prevent adverse chemical interactions between the excipients and the active ingredient. Finally, the biological activity of the active ingredient should not be seriously compromised.

It is thus particularly difficult to provide sufficient stability of this type of cytotoxic active ingredient in solid oral forms. Vinorelbine ditartrate in freeze-dried form should actually be kept in a sealed package, under an inert atmosphere and at a temperature below minus 15°C.

A compromise should be found, upon selecting the excipients, between stability and availability of the active ingredient. The excipients should protect the latter while not being an obstacle to its dissolution and its rapid availability to the organism.

Further, the health and environment risks related to the handling of cytotoxic compounds as a powder on an industrial scale, raise an additional problem which needs to be overcome.

The object of the present invention is therefore to end up with a solid pharmaceutical form with a shelf life of at least 24 months under storage conditions which do not require freezing, while having a bioavailability equivalent to that of the "soft capsule" form present on the market.

The oral forms based on vinorelbine ditartrate according to the present invention are solid forms made from conventional excipients for compression or for gelatine capsules (diluents, binders, disintegrating agents, flow agents, lubricants). Surprisingly, these forms have sufficient stability so as to be kept at 5°C in a sealed package for 24 months.

With the present invention, it is therefore possible to improve the comfort of the patient since gelatin capsule and tablet oral forms according to the invention allow home medication. They therefore contribute to observance of the treatment by the patient.

Further, with access to solid oral forms of the gelatine capsule or tablet type, production costs may be reduced as compared with technologies requiring that the active ingredient be maintained in solution or in a dispersion inside the pharmaceutical form (for example of the soft capsule type).

The oral forms according to the invention are solid forms made from mixtures of a water-soluble vinorelbine salt, namely vinorelbine ditartrate, and excipients. They consist of at least one diluent and one lubricant and may be obtained from several industrial manufacturing methods. These methods are standard methods for making solid forms, known to one skilled in the art.

The method for manufacturing the compositions according to the invention may consist in dry mixing the different components followed by distributing them into gelatin capsules or by compressing them with a final step for film-coating the tablets.

Thus, in the case of making them by direct dry mixing, the active ingredient is, in a first phase, mixed with the diluent(s), optionally the disintegrating agent as well as the flow agent, in a standard mixer of the pharmaceutical industry such as a tumbler mixer for example. This premix is stirred at room temperature for about ten minutes until a homogenous mixture is obtained. Subsequently, the lubricant is added into the mixer before proceeding with lubrication by continuous stirring for about 5 to 10 further minutes. The thereby obtained mixture may be used for filling gelatine capsules with adequate equipment of the pharmaceutical industry. Alternatively said mixture may also be compressed on a press in order to obtain tablets. In the latter case, the obtained tablets may advantageously be submitted to a film-coating step.

The mixtures may also be wet- or dry-granulated before a lubrication phase, so as to be in the same way as earlier, distributed into gelatin capsules or tablets before being possibly film-coated.

Thus, in the case of the making by wet granulation, the active ingredient is in a first phase, intimately mixed with the diluent and binder by means of a standard mixer of the pharmaceutical industry. Advantageously, this mixture is accomplished by means of a mixer-granulator-drier in order to avoid handling of the product on several pieces of equipment, considering the cytotoxic nature of the active ingredient.

Actual granulation may be achieved by adding a granulation solvent. The granulation solvent may be aqueous, alcoholic or hydro-alcoholic. In the case of the application of an alcoholic solvent, ethanol will be preferred as an alcoholic solvent and in the case of a hydro-alcoholic solvent system, this will be a water/ethanol mixture in a weight ratio comprised between 70/30 and 30/70, advantageously between 60/40 and 40/60, and more advantageously of 50/50. Preferentially and in order to provide maximum stability of the active ingredient, both during the granulation step and during storage, selection of an alcoholic solvent, in particular ethanol, is preferred.

The weight ratio of the granulation solvent relatively to the amount of mixture to be granulated may be comprised between 8 and 20%, preferentially between 10 and 25%. The thereby moistened mixture is stirred and mixed before proceeding with granulation, i.e. agglomeration of the ingredients as granules.

The granulated mixture then undergoes a drying step in order to obtain a dry grain, i.e. having a humidity of the order of that corresponding to the mixture before granulation. The drying may be carried out inside a mixer-granulator-drier with application of vacuum, this in order to avoid too high temperatures detrimental to the stability of the active ingredient. Alternatively, the granulated mixture may be dried in an oven to which a vacuum may be applied, or even in a fluidized air bed.

In a particular embodiment of the method, the obtained granules are calibrated to a size comprised between 100 and 250 µm, preferentially about 200 µm.

An external phase comprising a disintegrating agent and/or a flow agent may be added to these granules obtained via a wet route. The whole is intimately mixed in a mixer such as a tumbler mixer.

The lubricant is then always added to the inside of the mixer in order to obtain lubricated granules.

The lubricated granules may be placed into gelatin capsules or compressed on a tablet press according to techniques well known to one skilled in the art.

In the case of the making by dry granulation, the active ingredient is intimately mixed with the diluent, possibly with addition of a binder, in a mixer of the pharmaceutical industry. The mixture may then be granulated via a granulation step without any solvent being provided, such as for example by a slugging or even compacting step, on a roll compacter for example. The thereby obtained agglomerates may undergo a milling/calibrating step in order to reduce size and obtain granules.

In a particular embodiment of the method, the obtained granules are calibrated to a size comprised between 100 and 250 µm, preferentially about 200 µm.

As previously, an external phase comprising a flow agent and/or a disintegrating agent may be added to these granules.

The thereby obtained granules are mixed with a lubricant, during the time required for obtaining a uniform distribution of the lubricant on the granules.

The lubricated granules may, in the same way as previously, be placed in gelatin capsules or tablets on a tablet press, according to techniques well known to one skilled in the art.

Alternatively, the compositions according to the present invention may be obtained by direct dry mixing, i.e. direct intimate mixing of the active ingredient with a diluent and possibly a disintegrating agent.

The lubricant and a possible flow agent are lastly added at the end of mixing before distribution into gelatin capsules or compression as tablets.

The invention therefore relates to a stable pharmaceutical composition comprising a water-soluble vinorelbine salt, which is vinorelbine ditartrate, and at least one diluent and one lubricant, characterized in that it appears in a solid form intended for oral administration.

In the sense of the present invention, it is understood that a composition termed "stable" is one for which, after storage, optionally under an inert atmosphere, for a period of 24 months at a temperature comprised between 0 and 10°C, advantageously between 2°C and 8°C, the impurity content is less than 2%, advantageously less than 1% and even more preferentially less than 0.5%.

By the term of "diluent" is meant in the sense of the present invention, a substance with which the bulk of a pharmaceutical composition may be increased in order to provide bulk uniformity and ingredient active content uniformity of the final pharmaceutical form, a tablet or a gelatin capsule. Diluents further allow during the manufacturing processes a proper flow of the mixture for the active ingredients which generally do not flow properly. They also allow compression to be facilitated during the making of tablets.

By the term of "lubricant", in the sense of the present invention, is meant a substance capable or reducing friction between the different constituents of the excipient mixture as a powder, and possibly containing the active ingredient. They also provide a reduction in the adherence of the powder to the punch and the die. They also provide better transmission of the compression forces. However, when they are in excess, they reduce the cohesion of the tablets.

By the term of "binder", in the sense of the present invention, is meant a substance capable of promoting interparticular bonds. With the binders, it is possible to reduce the required compression force for obtaining tablets. Certain binders, such as cellulose derivatives, generate entanglement between the particles to be agglomerated. Others have a not very high melting point and, upon a rise of temperature induced during compression, are capable of forming interparticular bridges.

By the term of "flow agent", in the sense of the present invention, is meant a substance capable of improving the flow of a solid mixture by improving the fluidity of the powder and therefore the regular filling of the compression chamber.

By the term of "disintegrating agent", in the sense of the present invention, is meant a substance allowing the solid pharmaceutical form to disintegrate in the presence of liquid, for example in the stomach or upon contact with digestive fluids, thereby releasing the active ingredient.

By "film-coating agent", in the sense of the present invention, is meant a substance, most often a polymeric substance, capable of covering tablets, granules or even the gelatin capsule envelope with a fine film. It may play the role of a coloring agent or be used for hiding a taste or an unpleasant smell. It may also protect the patient or the medical personnel, at a manual and bucco-pharyngeal level, from the toxicity of an active ingredient. It may also be gastro-resistant or causing dialysis. In this case this is then called a coating agent because the amount to be deposited is larger.

By "film-coating adjuvant", in the sense of the present invention, is meant plasticizers with which the coating film may be prevented from being too brittle. They may also allow a reduction in the temperature for forming the film.

The diluent disclosed in the present description is sugars, advantageously sucrose, fructose, glucose, polyols, advantageously mannitol, xylitol, sorbitol, maltitol, lactitol, polysaccharides advantageously native or pre-gelatinized starch, maltodextrins, cyclodextrins, mineral compounds, advantageously dicalcium or tricalcium phosphate either dihydrates or anhydrous, cellulose derivatives, preferably microcrystalline cellulose, lactoses either monohydrates or anhydrous, as well as their mixtures, and is more advantageously dicalcium phosphate dihydrate, mannitol, pre-gelatinized maize starch, microcrystalline cellulose and their mixtures.

The lubricant disclosed in the present description is the salts of fatty acids, advantageously magnesium stearate, aluminum stearate, calcium stearate, sodium stearate, sorbitan stearate, zinc stearate, fatty acid esters, advantageously glycerol behenate, glycerol monostearate, glycerol palmitostearate, stearic acid, stearyl alcohol, castor oils, either hydrogenated or not, hydrogenated vegetable oils, maize oil, sodium benzoate, talc, sodium stearyl fumarate, fatty acid triglycerides, polyethylene glycol and its derivatives, as well as their mixtures.

The lubricant according to the invention is magnesium stearate.

The diluent according to the invention consists in a mixture of microcrystalline cellulose and of a constituent selected from D-mannitol, maize starch and dicalcium phosphate dihydrate.

The diluent proportion is comprised between 30 and 60%, more advantageously equal to about 56%.

The lubricant proportion is advantageously comprised between 0.5 and 10% of the total weight of the composition, advantageously between 1% and 5%, and still more advantageously equal to about 0.5%.

The composition according to the invention comprises a binder.

The binder disclosed in the present description is cellulose derivatives, advantageously hydroxypropylmethyl cellulose, hydroxypropyl cellulose, hydroxyethyl cellulose, methyl cellulose, cellulose, from polyvinylpyrrolidone, gums, advantageously guar gum, tragacanth gum, gum arabic, xanthan gum, sugars, sucrose or glucose, gelatin, polyethylene glycols, or a copolymer of vinylpyrrolidone and vinyl acetate, as well as their mixtures.

The binder according to the invention is polyvidone K30.

The binder proportion is comprised between 1 and 10% of the total weight of the composition, advantageously equal to about 2.5%.

The composition according to the invention comprises a disintegrating agent.

The disintegrating agent disclosed in the present description is sodium croscarmellose, sodium carmellose, calcium carmellose, cellulose, starch derivatives, preferably carboxymethyl starch, pre-gelatinized starches, native starches, polyvinylpyrrolidone derivatives, advantageously crospovidone or copovidone, as well as their mixtures, and advantageously is crospovidone, sodium carboxymethyl starch or sodium lacroscarmellose.

The disintegrating agent according to the invention is sodium croscarmellose.

The proportion of disintegrating agent is comprised between 1 and 10% of the total weight of the composition, advantageously between 2 and 8%, and more advantageously equal to about 5%.

The composition according to the invention comprises a flow agent.

The flow agent disclosed in the present description is hydrophilic or hydrophobic colloidal silicas, either hydrates thereof or anhydrous.

The flow agent according to the invention is hydrophilic colloidal silica dihydrate.

The total proportion of flow agent and/or of lubricant is comprised between 0.2 and 5% of the total weight of the composition, advantageously equal to about 0.75%.

According to an advantageous embodiment of the invention, the composition comprises by weight:
- about 56% of a diluent according to the invention, advantageously about 22% of microcrystalline cellulose and about 34% of pre-gelatinized maize starch or dicalcium phosphate dihydrate or D-mannitol;
- about 2.5% of binder, which is polyvidone K30;
- about 5% of disintegrating agent, which is sodium croscarmellose;
- about 0.25% of flow agent, which is colloidal silica dihydrate;
- about 0.5% of lubricant, which is magnesium stearate.

The composition according to the invention may appear as a powder or as a granule.

Alternatively, the composition according to the invention may be compacted into a tablet.

The composition according to the invention as a powder or granule may be distributed in a polymeric gelatin capsule, preferably selected from gelatin, hydroxypropylmethyl cellulose, and pullulan.

The gelatin capsule may also further comprise a coloring agent, advantageously selected from pigments and oxides as well as their mixtures, more advantageously selected from titanium oxides and iron oxides, and their mixtures.

The gelatin capsule advantageously comprises gelatin, iron oxides and titanium dioxide.

A film-coating agent may be deposited at the surface of the tablet.

The film-coating agent is advantageously selected from derivatives of cellulose, advantageously hydroxypropylmethyl cellulose, hydroxyethyl cellulose, hydroxypropyl cellulose, methyl cellulose, ethyl cellulose, cellulose acetate, sodium carmellose, acrylic derivatives, advantageously polybutyl methacrylate, poly 2-méthylaminoethyl methacrylate, polymethyl methacrylate, polyethyl acrylate, trimethylaminoethyl methacrylate chloride, cetyl alcohol, glycerol behenate, waxes, advantageously beeswax, carnauba wax, gelatin, shellac, coconut oil, hydrogenated castor oil, polyvinyl alcohol, polyvinylmethylether, polyvinyl acetate as well as their mixtures.

The film-coating agent proportion is advantageously comprised between 0.1 and 20% of the total weight of the tablet, advantageously between 0.5 and 10%.

The composition may further comprise at least one film-coating adjuvant.

The film-coating adjuvant is advantageously selected from polyoxyethylene and alkyl ethers, plasticizers, advantageously triethyl citrate, dibutyl sebacate, dibutyl phthalate, mygliol, triacetin, fillers, advantageously talc, silicas, titanium dioxide, coloring agents as well as their mixtures.

The film-coating adjuvant proportion is comprised between 0.01 and 5% of the total weight of the tablet.

In an advantageous embodiment of the invention, the composition comprises by weight about: 0.19% of polyethylene glycol, 0.81% of titanium dioxide, 0.01% of yellow quinoline colouring agent and 0.01% of red iron oxide.

The composition according to the invention comprises by weight from 30 to 50% of a water-soluble vinorelbine salt.

When the composition according to the invention is obtained by granulation, it advantageously comprises by weight from 30 to 50% of a water-soluble vinorelbine salt, still more advantageously about 35%.

When the composition according to the invention is obtained by dry mixing, it advantageously comprises by weight from 35 to 50% of a water-soluble vinorelbine salt, still more advantageously about 50%.

The water-soluble vinorelbine salt is vinorelbine ditartrate.

The composition according to the invention may advantageously be stored in a sealed package in order to increase its stability.

The composition according to the invention, compacted into tablets or distributed in a gelatin capsule, may thus be stored in a sealed package, preferably in a thermoformed blister coated with an air-proof and moisture-proof aluminized laminate or co-laminate according to techniques known to one skilled in the art.

The invention will now be illustrated in a nonlimiting way by the following examples.

Tablets and gelatin capsules according to the invention were prepared. Their stability as well as their dissolution rate were measured.

### Example No.1

A 35% vinorelbine ditartrate concentrated mixture was prepared by wet granulation, and an external phase (sodium croscarmellose, colloid silica dihydrate) was then added, before a step for lubrication and distribution in gelatin capsules. The obtained gelatin capsules were dosed with 30 mg of vinorelbine base.

The majority diluent is pre-gelatinized maize starch.

### 1.1. Unitary and centesimal formulations of the granules

| Components | Dosage with 30 mg of base | |
|---|---|---|
| | Weight in mg | % |
| Vinorelbine ditartrate | 41.55 | 35.00 |
| Pre-gelatinized maize starch | 40.42 | 34.05 |
| Microcrystalline cellulose | 26.94 | 22.70 |
| Polyvidone K30 | 2.97 | 2.50 |
| Sodium croscarmellose | 5.94 | 5.00 |
| Colloidal silica dihydrate | 0.30 | 0.25 |
| Magnesium stearate | 0.59 | 0.50 |
| Total | 118.71 | 100.000 |
| Gelatin capsule of size 3 | 1 gelatin capsule | / |

### 1.2. Stability test at 5°C in a sealed package after 6 and 12 months

The compositions as gelatin capsules are housed in a sealed package (T0) and stored at 5°C with an external 20% humidity rate. Next, the impurity content (in % of the total weight of the composition) is dosed after 6 and 12 months (T6 and T12 respectively) and compared to that dosed beforehand at T0. The difference between the percentages at T6 and T0 and then T12 and T0 indicate the change in the impurity content.

It is compared with that obtained for freeze-dried vinorelbine ditartrate stored under the same conditions.

The results are transferred into the table herein below. This operating procedure was applied in the following Examples 2 to 5.

| | Vinorelbine ditartrate | Gelatin capsules |
|---|---|---|
| Change in impurity content | | T0 to T6 : +0.5 |
| | T0 to T12 : +0.79 | T0 to T12 : +0.06 |

### 1.3. Dissolution test at T0

The amount of dissolved active ingredient is measured according to a standard European pharmacopeia protocol, in 1 liter of 0.1N HCl for a gelatin capsule at 37°C under 75 rpm stirring. The results are transferred into the table below. This operating procedure was applied in the following Examples 2 to 5.

| Time (min) | 0 | 5 | 10 | 15 | 30 | 45 |
|---|---|---|---|---|---|---|
| Amount of dissolved active ingredient (%) | 0 | 86 | 94 | 94 | 95 | 95 |

In order to evaluate bioavailability of vinorelbine ditartrate in the oral forms according to the invention, the dissolution kinetics of a commercial soft gelatin type capsule Navelbine Oral® (WO 03/101383), containing a vinorelbine ditartrate solution are indicated below (identical conditions, 50 rpm stirring).

| Time (min) | 0 | 5 | 10 | 15 | 30 | 45 |
|---|---|---|---|---|---|---|
| Amount of dissolved active ingredient (%) | 0 | 6 | 90 | 95 | 96 | 98 |

### Example No.2

A mixture with a 35% concentration of vinorelbine ditartrate was prepared by wet granulation and an external phase was then added (sodium croscarmellose, colloidal silica dihydrate) before a lubrication and gelatin capsule distribution step. The obtained gelatin capsules are dosed with 30 mg of vinorelbine base.

The majority diluent is dicalcium phosphate dihydrate.

### 2.1. Unitary and centesimal forms

| Components | Dosage with 30 mg of base | |
|---|---|---|
| | Weight in mg | % |
| Vinorelbine ditartrate | 41.55 | 35.00 |
| Dicalcium phosphate dihydrate | 40.42 | 34.05 |
| Microcrystalline cellulose | 26.94 | 22.70 |
| Polyvidone K30 | 2.97 | 2.50 |
| Sodium croscarmellose | 5.94 | 5.00 |
| Colloidal silica dihydrate | 0.30 | 0.25 |
| Magnesium stearate | 0.59 | 0.50 |
| Total | 118.71 | 100.000 |
| Gelatin capsules of size 3 | 1 gelatin capsule | / |

### 2.2. Stability test at 5°C in a sealed package after 6 and 12 months

| Change in the total amount of impurities |
|---|
| T0 to T6 : +0.18 |
| T0 to T12 : +0.07 |

### 2.3. Dissolution test at T0

| Time (min) | 0 | 5 | 10 | 15 | 30 | 45 |
|---|---|---|---|---|---|---|
| Amount of dissolved active ingredient (%) | 0 | 55 | 85 | 94 | 95 | 95 |

### Example No.3

A mixture with a 35% concentration of vinorelbine ditartrate was prepared by wet granulation, and then an external phase was added (sodium croscarmellose, colloidal silica dihydrate), before a step for lubrication and distribution in gelatin capsules. The obtained gelatin capsules are dosed with 30 mg of vinorelbine base.

The majority diluent is D-mannitol.

### 3.1. Unitary and centesimal formulations

| Components | Dosage with 30 mg of base | |
|---|---|---|
| | Weight in mg | % |
| Vinorelbine ditartrate | 41.55 | 35.00 |
| D-mannitol | 40.42 | 34.05 |
| Microcrystalline cellulose | 26.94 | 22.70 |
| Polyvidone K30 | 2.97 | 2.50 |
| Sodium croscarmellose | 5.94 | 5.00 |
| Colloidal silica dihydrate | 0.30 | 0.25 |
| Magnesium stearate | 0.59 | 0.50 |
| Total | 118.71 | 100.000 |
| Gelatin capsule of size 3 | 1 gelatin capsule | / |

### 3.2. Stability test at 5°C in a sealed package after 6 and 12 months

| Change in the total amount of impurities |
|---|
| T0 to T6 : +0.25 |
| T0 to T12 : +0.12 |

### 3.3. Dissolution test at T0

| Time (min) | 0 | 5 | 10 | 15 | 30 | 45 |
|---|---|---|---|---|---|---|
| Amount of dissolved active ingredient (%) | 0 | 84 | 94 | 94 | 95 | 95 |

### Example No.4

A mixture with a 35% concentration of vinorelbine ditartrate was prepared by dry mixing, before a step for lubrication and distribution in gelatin capsules. The obtained gelatin capsules are dosed with 30 mg of vinorelbine base.

The majority diluent is pre-gelatinized maize starch.

### 4.1. Unitary and centesimal formulations

| Components | Dosage with 30 mg of base | |
|---|---|---|
| | Weight in mg | % |
| Vinorelbine ditartrate | 41.55 | 35.00 |
| Pre-gelatinized maize starch | 40.42 | 34.05 |
| Microcrystalline cellulose | 26.94 | 22.70 |
| Polyvidone K30 | 2.97 | 2.50 |
| Sodium croscarmellose | 5.94 | 5.00 |
| Colloidal silica dihydrate | 0.30 | 0.25 |
| Magnesium stearate | 0.59 | 0.50 |
| Total | 118.71 | 100.000 |
| Gelatin capsule of size 3 | 1 gelatin capsule | / |

### 4.2. Stability tests at 5°C in a sealed package after 6 and 12 months

| Change in the total amount of impurities |
|---|
| T0 to T6 : +0.08 |
| T0 to T12 : +0.12 |

### 4.3. Dissolution test at T0

| Time (min) | 0 | 5 | 10 | 15 | 30 | 45 |
|---|---|---|---|---|---|---|
| Amount of dissolved active ingredient (%) | 0 | 77 | 100 | 100 | 100 | 100 |

### Example No.5

A mixture with a 35% concentration of vinorelbine ditartrate was prepared by wet granulation, and an external phase (sodium croscarmellose, colloidal silica dihydrate) was added, before a tablet lubrication and compression step. The obtained tablets are dosed with 30 mg of vinorelbine base.

The majority diluent is pre-gelatinized maize starch.

### 5.1. Unitary and centesimal formulations

| Components | Dosage with 30 mg of base | |
|---|---|---|
| | Weight in mg | % |
| Vinorelbine ditartrate | 41.55 | 35.00 |
| Pre-gelatinized maize starch | 40.42 | 34.05 |
| Microcrystalline cellulose | 26.94 | 22.70 |
| Polyvidone K30 | 2.97 | 2.50 |
| Sodium croscarmellose | 5.94 | 5.00 |
| Colloidal silica dihydrate | 0.30 | 0.25 |
| Magnesium stearate | 0.59 | 0.50 |
| Total | 118.71 | 100.000 |

The experiments above demonstrate that the compositions according to the invention are stable at 5°C in a sealed package for a minimum period of 12 months. A shelf life of 24 months at 5°C may therefore be reasonably contemplated for the compositions.

These experiments demonstrate that with the compositions according to the invention, more than 80% of their active ingredient may be released *in vitro* in less than 30 minutes.

The results of the dissolution experiments of compositions according to the invention have shown that after 30 minutes, the amount of dissolved vinorelbine ditartrate is identical with that observed in the case of a soft capsule of commercial Navelbine Oral® (described in WO 03/101383). Observation of the same bioavailability of vinorelbine ditartrate after 30 minutes may therefore be expected for both of these pharmaceutical forms.

### Reference Example No.6

A mixture with a 35% concentration of vinorelbine ditartrate was prepared by dry mixing, before a step for lubrication and distribution in gelatin capsules.

Vinorelbine ditartrate is incorporated into the diluents (pregelatinized maize starch and microcrystalline cellulose), to the disintegrating agent (sodium croscarmellose) and to the flow agent (colloidal silica dihydrate) in a mixer and preferentially a tumbler mixer. The majority diluent is pregelatinized maize starch majoritaire. Mixing is carried out during a period for obtaining a homogenous mixture of the different components, this duration is preferentially 10 minutes. The mixing step is followed by a lubrication step carried out by the same mixer with magnesium stearate as lubricant. The duration of this step for obtaining optimum lubrication of the mixture is preferentially 5 minutes. The lubrication step is followed by distributing the mixture into gelatine capsules. The obtained gelatine capsules are dosed with 30 mg of vinorelbine base.

### 6.1. Unitary and centesimal formulations

| | Dosage with 30 mg of base | |
|---|---|---|
| Components | Weight in mg | Weight in mg |
| Vinorelbine ditartrate | 41.55 | 35.00 |
| Pre-gelatinized maize starch | 43.39 | 36.55 |
| Microcrystalline cellulose | 26.94 | 22.70 |
| Sodium croscarmellose | 5.94 | 5.00 |
| Colloidal silica dihydrate | 0.30 | 0.25 |
| Magnesium stearate | 0.59 | 0.50 |
| Total | 118.71 | 100.000 |
| Gelatin capsule of size 3 | 1 gelatin capsule | / |

## Claims

1. A stable pharmaceutical composition comprising by weight from 30 to 50% of vinorelbine ditartrate and
- 30 to 60% of a diluent of the total weight of the composition, the diluent consisting in a mixture of microcrystalline cellulose and of a constituent selected from D-mannitol, maize starch and dicalcium phosphate dihydrate,
- magnesium stearate and hydrophilic colloidal silica dihydrate, **characterized in that** the total hydrophilic colloidal silica dihydrate and/or magnesium stearate proportion is comprised between 0.2 and 5% of the total weight of the composition,
- 1 to 10% of polyvidone K30 of the total weight of the composition, and
- 1 to 10% of sodium croscarmellose of the total weight of the composition,
**characterized in that** it appears in a solid form intended for oral administration.

2. The composition according to the preceding claim, **characterized in that** the diluent proportion is equal to about 56% of the total weight of the composition.

3. The composition according to any of the preceding claims, **characterized in that** the magnesium stearate proportion is comprised between 0.5 and 5% of the total weight of the composition.

4. The composition according to any of the preceding claims, **characterized in that** the magnesium stearate proportion is equal to about 0.5% of the total weight of the composition.

5. The composition according to any of the preceding claims, **characterized in that** the polyvidone K30 proportion is equal to about 2.5% of the total weight of the composition.

6. The composition according to any of the preceding claims, **characterized in that** the sodium croscarmellose proportion is comprised between 2 and 8% of the total weight of the composition.

7. The composition according to any of the preceding claims, **characterized in that** the sodium croscarmellose proportion is equal to 5% of the total weight of the composition.

8. The composition according to any of the preceding claims, **characterized in that** the total hydrophilic colloidal silica dihydrate and/or magnesium stearate proportion is equal to about 0.75% of the total weight of the composition.

9. The composition according to any of the preceding claims, **characterized in that** it comprises by weight:
- about 56% of the diluent;
- about 2.5% of polyvidone K30;
- about 5% of sodium croscarmellose;
- about 0.25% of colloidal silica dihydrate;
- about 0.5% of magnesium stearate.

10. The composition according to claim 9, **characterized in that** it comprises by weight about 22% of microcrystalline cellulose and about 34% of pre-gelatinized maize starch or dicalcium phosphate dihydrate or D-mannitol.

11. The composition according to any of the preceding claims, **characterized in that** it appears as a powder or granule or as a powder or granule distributed in a polymeric gelatin capsule,or is compacted into a tablet.

12. The composition according to claim 11, **characterized in that** the polymeric gelatin capsule is selected from gelatin, hydroxypropylmethyl cellulose and pullulan.

13. The composition according to any of claims 1 to 12, **characterized in that** it comprises by weight about 35% of vinorelbine ditartrate.

## Patentansprüche

1. Stabile pharmazeutische Zusammensetzung, umfassend von 30 bis 50 Gew% Vinorelbinditartrat und
- 30 bis 60 Gew% eines Lösemittels des Gesamtgewichts der Zusammensetzung, wobei das Lösemittel aus einem Gemisch aus mikrokristalliner Cellulose und einem Bestandteil besteht, ausgewählt aus D-Mannitol, Maisstärke und Dicalciumphosphatdihydrat,
- Magnesiumstearat und hydrophiles kolloidales Siliciumdioxid-Dihydrat, **dadurch gekennzeichnet, dass** die gesamte hydrophile kolloidale Siliciumdioxid-Dihydrat- und/oder Magnesiumstearat-Proportion im Bereich zwischen 0,2 und 5 % des Gesamtgewichts der Zusammensetzung liegt,
- 1 bis 10 % Polyvidon K30 des Gesamtgewichts der Zusammensetzung und
- 1 bis 10 % Natriumcroscarmellose des Gesamtgewichts der Zusammensetzung,
**dadurch gekennzeichnet, dass** sie in einer festen Form vorliegt, die zur oralen Verabreichung vorgesehen ist.

2. Zusammensetzung nach dem vorhergehenden Anspruch, **dadurch gekennzeichnet, dass** die Lösemittel-Proportion gleich ungefähr 56 % des Gesamtgewichts der Zusammensetzung ist.

3. Zusammensetzung nach einem der vorhergehenden Ansprüche, **dadurch gekennzeichnet, dass** die Magnesiumstearat-Proportion im Bereich zwischen 0,5 und 5 % des Gesamtgewichts der Zusammensetzung liegt.

4. Zusammensetzung nach einem der vorhergehenden Ansprüche, **dadurch gekennzeichnet, dass** die Magnesiumstearat-Proportion gleich ungefähr 0,5 % des Gesamtgewichts der Zusammensetzung ist.

5. Zusammensetzung nach einem der vorhergehenden Ansprüche, **dadurch gekennzeichnet, dass** die Polyvidon K30-Proportion gleich ungefähr 2,5 % des Gesamtgewichts der Zusammensetzung ist.

6. Zusammensetzung nach einem der vorhergehenden Ansprüche, **dadurch gekennzeichnet, dass** die Natriumcroscarmellose-Proportion im Bereich zwischen 2 und 8 % des Gesamtgewichts der Zusammensetzung liegt.

7. Zusammensetzung nach einem der vorhergehenden Ansprüche, **dadurch gekennzeichnet, dass** die Natriumcroscarmellose-Proportion gleich 5 % des Gesamtgewichts der Zusammensetzung ist.

8. Zusammensetzung nach einem der vorhergehenden Ansprüche, **dadurch gekennzeichnet, dass** die gesamte hydrophile kolloidale Siliciumdioxid-Dihydrat- und/oder Magnesiumstearat-Proportion gleich ungefähr 0,75 % des Gesamtgewichts der Zusammensetzung ist.

9. Zusammensetzung nach einem der vorhergehenden Ansprüche, **dadurch gekennzeichnet, dass** sie Folgendes umfasst:
- ungefähr 56 Gew% des Lösemittels;
- ungefähr 2,5 Gew% Polyvidon K30;
- ungefähr 5 Gew% Natriumcroscarmellose;
- ungefähr 0,25 Gew% kolloidales Siliciumdioxid-Dihydrat;
- ungefähr 0,5 Gew% Magnesiumstearat.

10. Zusammensetzung nach Anspruch 9, **dadurch gekennzeichnet, dass** sie ungefähr 22 Gew% mikrokristalline Cellulose und ungefähr 34 Gew% vorgelatinierte Maisstärke oder Dicalciumphosphatdihydrat oder D-Mannitol umfasst.

11. Zusammensetzung nach einem der vorhergehenden Ansprüche, **dadurch gekennzeichnet, dass** sie in Form eines Pulvers oder Granulats oder als eines Pulvers oder Granulats, das in einer polymeren Gelatinekapsel verteilt ist, oder in einer Tablette verdichtet vorliegt.

12. Zusammensetzung nach Anspruch 11, **dadurch gekennzeichnet, dass** die polymere Gelatinekapsel ausgewählt ist aus Gelatine, Hydroxypropylmethylcellulose und Pullulan.

13. Zusammensetzung nach einem der Ansprüche 1 bis 12, **dadurch gekennzeichnet, dass** sie ungefähr 35 Gew% Vinorelbinditartrat umfasst.

## Revendications

1. Composition pharmaceutique stable comprenant en poids de 30 à 50 % de ditartrate de vinorelbine et
- 30 à 60 % d'un diluant du poids total de la composition, le diluant consistant en un mélange de cellulose microcristalline et d'un constituant choisi parmi le D-mannitol, l'amidon de maïs et le phosphate dicalcique dihydraté,
- du stéarate de magnésium et de la silice colloïdale dihydratée hydrophile, **caractérisée en ce que** la proportion totale de silice colloïdale dihydratée hydrophile et/ou de stéarate de magnésium est comprise entre 0,2 et 5 % du poids total de la composition,
- 1 à 10 % de polyvidone K30 du poids total de la composition, et
- 1 à 10 % de croscarmellose sodique du poids total de la composition,
**caractérisée en ce qu'**elle apparaît sous une forme solide destinée à une administration orale.

2. Composition selon la revendication précédente, **caractérisée en ce que** la proportion du diluant est égale à environ 56 % du poids total de la composition.

3. Composition selon l'une quelconque des revendications précédentes, **caractérisée en ce que** la proportion de stéarate de magnésium est comprise entre 0,5 et 5 % du poids total de la composition.

4. Composition selon l'une quelconque des revendications précédentes, **caractérisée en ce que** la proportion de stéarate de magnésium est égale à environ 0,5 % du poids total de la composition.

5. Composition selon l'une quelconque des revendications précédentes, **caractérisée en ce que** la proportion de polyvidone K30 est égale à environ 2,5 % du poids total de la composition.

6. Composition selon l'une quelconque des revendications précédentes, **caractérisée en ce que** la proportion de croscarmellose sodique est comprise entre 2 et 8 % du poids total de la composition.

7. Composition selon l'une quelconque des revendications précédentes, **caractérisée en ce que** la proportion de croscarmellose sodique est égale à 5 % du poids total de la composition.

8. Composition selon l'une quelconque des revendications précédentes, **caractérisée en ce que** la proportion totale de silice colloïdale dihydratée hydrophile et/ou de stéarate de magnésium est égale à environ 0,75 % du poids total de la composition.

9. Composition selon l'une quelconque des revendications précédentes, **caractérisée en ce qu'**elle comprend en poids :
- environ 56 % du diluant ;
- environ 2,5 % de polyvidone K30 ;
- environ 5 % de croscarmellose sodique ;
- environ 0,25 % de silice colloïdale dihydratée ;
- environ 0,5 % de stéarate de magnésium.

10. Composition selon la revendication 9, **caractérisée en ce qu'**elle comprend en poids environ 22 % de cellulose microcristalline et environ 34 % d'amidon de maïs prégélatinisé ou de phosphate dicalcique dihydraté ou de D-mannitol.

11. Composition selon l'une quelconque des revendications précédentes, **caractérisée en ce qu'**elle apparaît sous la forme d'une poudre ou de granule ou sous la forme d'une poudre ou de granule réparti dans une gélule en polymère, ou est compactée en un comprimé.

12. Composition selon la revendication 11, **caractérisée en ce que** la gélule en polymère est choisie parmi la gélatine, l'hydroxypropylméthylcellulose et le pullulan.

13. Composition selon l'une quelconque des revendications 1 à 12, **caractérisée en ce qu'**elle comprend en poids environ 35 % de ditartrate de vinorelbine.
